# EUROPEAN PATENT APPLICATION

(11) **EP 1 329 211 A1**
(43) Date of publication of application: **23.07.2003**
(21) Application number: 01972533.2
(22) Date of filing: 27.09.2001
(51) Int. Cl.: A61K 7/00, C08L 101/00, C08K 3/00

(54) **ANTIBACTERIAL RESIN**

(30) Priority: 06.10.2000 JP 2000307299
(71) Applicant: KABUSHIKI KAISHA SANGI, Tokyo 104-0045 (JP)
(72) Inventor: SAITO, Tomoki, c/o Kabushiki Kaisha Sangi, Tokyo 104-0045 (JP); NAKANAGA, Hiroshi, c/o Kabushiki Kaisha Sangi, Tokyo 104-0045 (JP)
(74) Representative: Fürniss, Peter, Dr.rer.nat.Dipl.Chem
(86) International application number: JP0108440
(87) International publication number: WO02030365

(57) **Abstract**

An antibacterial resin is allowed to contain an inorganic antibacterial agent so as to retain the quality of cosmetics and improve the applicability, where the antibacterial rein is of a mean particle size of 0.1 to 1,000 µm and is produced preferably by methods such as emulsion polymerization and suspension polymerization of a calcium phosphate-series antibacterial agent added to a monomer component of a resin.

## Description

### TECHNICAL FIELD

The present invention relates to an antibacterial resin for cosmetics.

### BACKGROUND OF THE INVENTION

For the purpose of the preparation of color tone, the adjustment of applicability such as spreadability and adhesiveness and gloss and the provision of shape retention for cosmetic preparations, traditionally, inorganic pigments such as mica, talc, kaolin, silicic anhydride, red oxide, yellow iron oxide, black iron oxide, ultramarine blue, Prussian blue, titanium oxide, zinc oxide and mica titanium have been blended.

However, these inorganic pigments aggregate together, via the interaction of the powder particles thereof, so that the dispersibility thereof in cosmetics may be reduced or these pigments may be deposited or separated out in cosmetics. Depending on the type, shape and particle size of a powder, the powder disadvantageously causes rough touch and problematic applicability such as poor smoothness or spreadability on skin. Thus, these inorganic pigments are not so preferable as powders for cosmetics.

For the purpose of the improvement of the applicability of cosmetics, alternatively, various synthetic resins prepared into the form of finely divided powder are blended in cosmetics, which include for example nylon powder, polyester powder, polyethylene powder, polymethacrylate methyl powder, silicone powder, polyacrylonitrile powder, and polypropylene powder.

These organic polymers (referred to as polymer hereinafter)-based finely divided powders have small specific gravities and are more elastic than inorganic pigments, so the finely divided powders are more preferable owing to the smoothness and softness during use. Thus, such finely divided powders are used in large amounts.

However, the polymer-based finely divided powders absorb organic antibacterial agents, so that the finely divided powders per se are readily modified, disadvantageously.

So as to keep the quality of cosmetics, generally, organic antibacterial agents such as benzoic acid, benzoate, and p-oxybenzoate ester are blended.

Particularly, p-oxybenzoate esters (p-oxybenzoate isobutyl, p-oxybenzoate isopropyl, p-oxybenzoate ethyl, p-oxybenzoate butyl, p-oxybenzoate propyl, etc.) are most frequently used, but such organic antibacterial agents are disadvantageously absorbed highly by nylon powder currently used at the highest level among the polymer-based finely divided powders.

Because the polymer-based finely divided powders absorb organic antibacterial agents and are then modified, as described above, the effect of the preservatives per se is blocked. Additionally, the improvement of the applicability of cosmetics is disturbed. Therefore, the organic antibacterial agents cannot exert any satisfactory effect.

Organic bacterial agents strongly irritate skin, causing unpleasant feeling. For the purpose of reducing or overcoming such disadvantages, therefore, an approach of applying inorganic antibacterial compositions such as zeolite to cosmetic compositions (Japanese Patent Laid-open Publication No. 1985 (Sho 60)-174707; Japanese Patent Laid-open Publication No. 2000-7520) and an approach of using inorganic antibacterial agents in combination with organic antibacterial agents such as p-oxybenzoate ester (Japanese Patent Laid-open Publication No. 1998 (Hei 10)-45563) are disclosed.

However, inorganic antibacterial agents when added at a small amount cannot sufficiently exert the antibacterial effect or the applicability of the resulting cosmetics is insufficient due to the inorganic antibacterial agents used.

### DISCLOSURE OF THE INVENTION

It is a purpose of the invention to provide a finely divided polymer particle capable of improving the applicability of cosmetics, while retaining the quality of cosmetics, as well as a method for producing the finely divided polymer particle.

The inventors made investigations so as to overcome the problems. Consequently, the inventors have found that the blending in cosmetics of a finely divided polymer particle with an antibacterial ceramics kneaded therein is effective for the retention of the quality of cosmetics and can improve the applicability. Thus, the invention has been achieved.

The antibacterial finely divided polymer particle of the invention can be produced by adding a calcium phosphate-based antibacterial agent to a monomer component of a resin and then treating the resulting mixture using methods such as emulsification polymerization and suspension polymerization.

First, the polymer composing the antibacterial finely divided polymer particle of the invention is now described. The synthetic polymer composing the finely divided polymer particle includes for example modified olefin resins such as ethylene-(meth)acrylate copolymer, maleic anhydride-modified polyethylene, maleic anhydride-modified polypropylene, and epoxy-modified polypropylene; styrenic resins such as polystyrene, styrene-(meth)acrylate alkyl ester copolymer, styrene-maleic anhydride copolymer, acrylonitrile-styrene copolymer, styrene-maleic anhydride copolymer, styrene-(meth)acrylate ester-(meth)acrylate copolymer, styrene-butadiene copolymer, and acrylonitrile-butadiene-styrene copolymer; acrylate resins such as (meth)acrylate-(meth)acrylate ester copolymer and (meth)acrylate ester [(meth)acrylate methyl, (meth)acrylate ethyl, etc.]; polyamides such as copolymerized polyamide resin and modified polyamide resin; thermoplastic resins such as thermoplastic polyurethane, polycarbonate, and polyamino acid; thermosetting resins such as unsaturated polyester resin, diallyl phthalate resin, silicone resin, epoxy resin, thermosetting polyurethane, phenol resin, and amino resin [polyurea, melamine resin, etc.]; aromatic vinyls such as styrene, α-methylstyrene, p-methylstyrene, vinyltoluene, isopropenylstyrene, and chlorostyrene; olefins such as ethylene, propylene, and isobutylene; halogenated vinyls such as vinyl chloride, vinylidene chloride, vinyl bromide, and vinyl fluoride; unsaturated alcohols such as (meth) allyl alcohol, and croton alcohol; vinyl esters of saturated monocarboxylic acids, such as vinyl acetate and vinyl propionate; (meth)allyl esters of saturated aliphatic monocarboxylic acid, such as allyl acetate and allyl propionate; and vinyl ethers such as methyl vinyl ether and butyl vinyl ether.

Then, the inorganic antibacterial agent composing the antibacterial finely divided polymer particle of the invention is now described.

The inorganic antibacterial agent giving an antibacterial effect to the antibacterial finely divided polymer particle of the invention contains an antibacterial metal or ion and a carrier immobilizing the metal or ion thereon.

The antibacterial metal immobilized on an inorganic antibacterial agent for use in the antibacterial finely divided polymer particle of the invention includes at least one selected from the group consisting of silver, copper, zinc, gold, platinum and nickel, from the respect of the safety profile for humans. From the respect of the retention of high antibacterial action, productivity and production cost, the use of silver, copper and zinc among the antibacterial metals is the most preferable. These antibacterial metals may be used singly or may be used in combination of plural species thereof.

Alternatively, the carrier immobilizing an antibacterial metal element or metal ion thereon includes at least one selected from the group consisting of alumina, silica gel, zeolite, phosphate salt-series compounds such as calcium phosphate and zirconium phosphate, calcium carbonate, calcium silicate, bentonite, and titanium oxide.

The compounds, namely alumina, silica gel, zeolite, phosphate salt-series compounds such as calcium phosphate and zirconium phosphate, calcium carbonate, calcium silicate, bentonite, and titanium oxide are safe for humans and have high immobilization potency of metal elements and/or metal ions. Among these carriers, a single compound is selected for use as such carrier, but plural compounds may also be selected for use as such carriers.

Among them, preferably, phosphate salt-series compounds are selected for use, because these compounds have high ion exchange potency and are substances causing the antibacterial metals immobilized thereon to be solubilized at a smaller amount. As to the form of such antibacterial metal immobilized on such carrier, further, it is suggested that not the entirety of the metal is ion exchanged to the metal ion but a part of the metal is adsorbed and retained. From the antibacterial respect, such form is preferable.

Specific examples of the phosphate salt-series compounds include at least one selected from the group consisting of calcium phosphate-series compounds such as tricalcium phosphate, calcium hydrogen phosphate, hydroxyapatite, calcium hydrogen pyrophosphate, and calcium pyrophosphate, titanate phosphate compounds, zirconium phosphate-series compounds, magnesium phosphate-series compounds, aluminium phosphate-series compounds, manganese phosphate-series compounds andiron phosphate-series compounds. The antibacterial agent containing such phosphate salt-series compound as the carrier causes a smaller amount of metal ions to be solubilized and can therefore sustain the antibacterial effect.

These carriers may be naturally occurring products or synthetic products. Synthetic products are preferable because particles of uniform quality can be recovered. In case of the synthesis of phosphate salts by wet method via solution reaction, amorphous phosphate salts can be produced. Further calcination process thereof can yield phosphate salts with higher crystallizability. Depending on the production method, phosphate salts of various crystallizability can be recovered. Any of the phosphate salts may be satisfactory. Additionally, phosphate salts containing crystal water may be satisfactory.

Among the phosphate salt-series compounds, further, calcium phosphate-series compounds are the most preferably used in particular because the compounds have good affinity (biological affinity) to humans, high antibacterial sustainability and excellent safety profile. Other than those described above, calcium phosphate-series compounds such as halogenated apatite Ca₁₀(PO₄)₆X₂(X=F, Cl) and non-stoichiometric apatite Ca_{10-z}(HPO₄)_{y}(PO₄)_{6-y}X_{2-y} · zH₂O (X=OH, F, Cl; y and z are at an inconstant ratio) may be satisfactory.

Among the compounds described as such carriers, in accordance with the invention, preferably, phosphate salt-series compounds, particularly calcium phosphate-series compounds are selected as the carriers; on the carriers is immobilized at least one antibacterial metal selected from silver, copper and zinc among the antibacterial metals, to prepare the inorganic antibacterial agents, preferably. The method for immobilizing the antibacterial metal on the carriers includes a method of immobilizing a metal element and/or a metal ion via adsorption, a method of immobilizing them via ion exchange reaction, or a method of immobilizing them via mechanochemical reaction.

Herein, the mechanochemical reaction means a method for producing slurry of an antibacterial agent of a uniform particle size, via adsorption and/or ion exchange from a starting material, using a mix unit such as ball mill. For example, a starting material (calcium-series compounds such as calcium carbonate, phosphoric acid, etc.) for producing a carrier and an aqueous solution of an antibacterial metal are placed in a ball mill, which is then operated for a given period of time, to allow the zirconia ball in the ball mill to agitate the slurry of the starting material and simultaneously pulverize the resulting reaction product. In such manner, the mechanochemical reaction is carried out for a given period of time, thereby progressing simultaneously the reaction of the starting material and the pulverization of the reaction product, so that a homogenous antibacterial agent of a uniform particle size can be recovered, which is preferable for mass-scale production.

As the inorganic antibacterial agent, the antibacterial metal is immobilized within a range of 0.01 % by weight to 30 % by weight, preferably 0.05 % by weight to 10.0 % by weight of the carrier. When the amount of the antibacterial metal to be immobilized is less than 0.01 % by weight, the antibacterial performance is low. Hence, the antibacterial agent should be used at a large amount. When the antibacterial metal is immobilized at an amount above 30.0 % by weight, the antibacterial metal is readily dissociated, because the association between some of the antibacterial metal and the carrier is low. Thus, the resulting cosmetic composition is likely to be colored.

Within a range not disturbing the object of the invention and depending on another object, additionally, other inorganic compounds for example silicone dioxide and zinc oxide may be contained in the inorganic antibacterial agent, other than the inorganic compound as the carrier and the antibacterial metal. For example, silicone dioxide has an effect of improving the whiteness of the antibacterial agent, while zinc oxide has an effect of improving the antibacterial spectrum (enlarging the range of the subject bacterial species covered with the antibacterial effect) of the antibacterial agent. Any of the inorganic compounds is safe for humans. In this case, the use of silver in particular as the antibacterial metal enlarges the subject range covered with the antibacterial action. Additionally, copper has an anti-fungal effect as well.

Further, preferably, inorganic antibacterial agents using phosphate salt-series compounds as the carriers are additionally subjected to calcination process at 500°C to 1200°C. Inorganic antibacterial agents treated with the calcination process are at a very low ratio of solubilized antibacterial metals and have far greater sustainability (durability) of the antibacterial effect, compared with those without such calcination process.

Still further, the particle size of the inorganic antibacterial agent is preferably 5 µm or less, more preferably 1 µm or less. When the particle size is above 5 µm, the specific surface area thereof is smaller. So as to allow the inorganic antibacterial agent to exert its antibacterial effect, therefore, the amount of the inorganic antibacterial agent to be blended should be increased. Due to the large particle size, then, the powder of the antibacterial agent is readily dissociated from the finely divided polymer particle allowed to contain the inorganic antibacterial agent. When the particle size of the inorganic antibacterial agent is 1 µm or less, the dissociation of the powder of the inorganic antibacterial agent from the polymer can be prevented, depending on the size of the finely divided polymer particle containing the inorganic antibacterial agent, so that the quality of the resulting cosmetics can be retained in a stable way.

The method for producing the antibacterial finely divided polymer particle of the invention is now described.

As described above, the antibacterial finely divided polymer particle of the invention can be produced by adding a calcium phosphate-series antibacterial agent to a monomer component of a resin, using methods such as emulsification polymerization, suspension polymerization and the like.

In case of emulsification polymerization, for example, a monomer mixture containing an inorganic antibacterial component and a polymerizable monomer is polymerized in the presence of an emulsifier and a water-soluble polymerization initiator in an aqueous medium, to produce the antibacterial finely divided polymer particle of the invention.

Otherwise, the antibacterial finely divided polymer particle of the invention can be produced by soap-free emulsification polymerization with no use of any emulsifier.

For polymerization of a monomer component, a polymerization initiator can be used. The polymerization initiator includes polymerization initiators traditionally known. The polymerization initiator includes radical polymerization initiators, for example hydrogen peroxide; persulfate salts such as sodium persulfate, ammonium persulfate, and potassium persulfate; organic peroxides such as benzoyl peroxide, lauroyl peroxide, caproyl peroxide, peracetic acid, t-butyl hydroxyperoxide, methyl ethyl ketone peroxide, and t-butyl perphthalate; and azo compounds such as azobisisobutyronitrile and azobisisobutylamide. These polymerization initiators may be used singly or in combination with two or more thereof in an appropriate mixture.

For progressing the polymerization, further, chain transfer agents such as lauryl mercaptan, dodecylmercaptan, 2-mercaptoethanol, 2-mercaptoacetic acid, carbon tetrachloride and carbon tetrabromide may be added, so as to adjust the molecular weight of the resulting resin. These may be used singly or in combination of two or more in an appropriate mixture.

Methods other than the method described above include a method of progressing polymerization after mixing an inorganic antibacterial component with a polymer intermediate, a method of mixing an inorganic antibacterial component with a polymer at a molten state at the termination of polymerization, and a method of mixing an inorganic antibacterial component with polymer pellets and subsequently molding the resulting mixture.

Furthermore, a method exists, including attaching the powder of an inorganic antibacterial agent onto the finely divided polymer particle, applying physical force using ball mill, sand mill, attritor, roll mill, hybridizer and the like to compress the inorganic antibacterial agent into the surface of the finely divided polymer particle to allow the finely divided polymer particle to contain the inorganic antibacterial agent.

The mean particle size of the antibacterial finely divided polymer particle is 0.1 µm to 1, 000 µm, preferably 0.1 µm to 50 µm. For use in cosmetics, the mean particle size thereof is more preferably about 1 µm to 20 µm.

The particle size of the finely divided polymer particle can be selected within the range, depending on the use. When a finely divided polymer particle of 20 µm or less is used, such finely divided polymer particle can be dispersed uniformly in a resin composition and can additionally increase the content of the antibacterial metal component in the resin composition, so that higher antibacterial effect can be recovered at a smaller amount of the antibacterial metal component added.

As to the particle size of the inorganic antibacterial agent, the upper limit thereof is 1/2-fold or less the particle size of the finely divided polymer particle containing the inorganic antibacterial agent. Additionally, an inorganic antibacterial agent of a finely divided powder of 1.0 µm or less is preferably used. When a finely divided polymer particle of a mean particle size of about 10 µm is used, for example, an inorganic antibacterial agent of a particle size of 5.0 µm or less should thus be used. Preferably, an inorganic antibacterial agent of a particle size of 1.0 µm is preferably used.

The content of the inorganic antibacterial agent is within a range of 0.01 to 50 % by weight, preferably 0.1 to 30 % by weight. When the content thereof is too less, the inorganic antibacterial agent has poor antibacterial action. When the content thereof is too much, the particle of the inorganic antibacterial agent contained in the finely divided polymer particle is readily dissociated from the finely divided polymer particle.

Depending on another purpose and within a range not disturbing the objects of the invention, herein, the antibacterial finely divided polymer particle of the invention may contain additive components such as various functional materials, for example ultraviolet reflector, ultraviolet absorbent, pigment, lipid adsorbent, moisturizer, and water-repellent oil-repellent substances, other than the inorganic antibacterial agent.

The ultraviolet reflector includes for example zirconium oxide and aluminium oxide. The ultraviolet absorbent includes for example titanium oxide and zinc oxide. The pigment includes for example talc, kaolin, mica, sericite, muscovite, synthetic mica, lepidolite, zinc yellow, silica, iron oxide, loess, chromium oxide, chromium hydroxide, and ultramarine blue. The lipid adsorbent includes for example hydroxyapatite, calcium phosphate compounds such as tricalcium phosphate, calcium hydrogen phosphate, and calcium pyrophosphate, zeolite and bentonite.

The moisturizer includes for example collagen powder and chitosan powder. The water-repellent oil-repellent substance includes for example silicone resins such as polymethylsilsesquioxane powder and methylpolysiloxane powder, and fluorine resin powders such as Teflon.

As the water-repellent oil-repellent substance, additionally, an inorganic powder of a particle size 1/2-fold or less the particle size of the finely divided polymer particle, which is insoluble or slightly soluble in water and oil or organic solvents such as ethanol, can be used after treatment for water-repellency and oil repellency. For example, finely divided particles of zirconium oxide, zinc yellow and the like described above are surface treated with fluorine compounds to prepare water-repellent oil-repellent powders.

Any general method for surface treating the finely divided particle with fluorine compounds may be satisfactory with no specific limitation. For example, a fluorine compound is dissolved or dispersed in water and/or a medium such as alcohol, acetone and toluene, and then, the finely divided particle is added to the resulting mixture. So as to mix together both the components well or so as to put both the components in contact together, the surface treatment is done, using mechanical units such as ball mill and vibration mill for agitation. By subsequently distilling off or filtering off the medium used for the treatment, the fluorine compound is coated on the surface of the finely divided particle.

The fluorine compound for use in the treatment of the powder surface for water-repellency and oil-repellency includes for example perfluoroalkylsilane, perfluoroalkylsilazane, perfluorodecalin, perfluorooctane, perfluoropentane, and perfluorodecane.

Herein, the amount of the fluorine compound used for the treatment is preferably at 0.1 to 30 % by weight of the powder. When the fluorine compound is less than 0.1 % by weight, the resulting water repellency and oil repellency are insufficient. When the fluorine compound is at 30 % by weight or more, the amount of the fluorine compound is at a supersaturated state, so that the applicability during use is deteriorated due to the excess of the fluorine compound.

The various additive components described above other than the inorganic antibacterial agent are used under the same conditions as those for the inorganic antibacterial agent. As the additive components, in other words, a powder of a mean particle size 1/2-fold or less the particle size of the finely divided polymer particle is used. The particle size is preferably 1/10-fold or less the particle size of the finely divided polymer particle. The additive components are contained at an amount such that the total of the inorganic antibacterial agent and the additive components is within a range of 50 % by weight or less of the finely divided polymer particle. When the amount is too much, the particle of the inorganic antibacterial agent or the particle of the additive component as contained in the finely divided polymer particle is readily dissociated from the finely divided polymer particle.

The method for allowing the additive components to be contained in the finely divided polymer particle is the same as in the case of the inorganic antibacterial agent.

The antibacterial finely divided polymer particle of the invention has great effects of retaining the quality of cosmetics and improving the applicability of cosmetics during use. Therefore, the antibacterial finely divided polymer particle is used at any form in cosmetics, satisfactorily, with no specific limitation. For example, the antibacterial finely divided polymer particle maybe applicable to skin in forms such as liquid, emulsion, cream, gel and powder.

The antibacterial finely divided polymer can be applied to cosmetic types, such as fundamental cosmetics such as cream, emulsion, lotion, essence, facial cleansing, and pack, makeup cosmetics such as eye shadow, cheek, lipstick, eyeliner, foundation, and liquid foundation, toiletry articles such as body shampoo, soap, bathing agent, and deodorant, hair care products such as shampoo, rinse, treatment, conditioner and hair cream or liquid, and oral care products such as tooth paste and mouth wash.

When the antibacterial finely divided polymer particle of the invention is to be blended in cosmetics, additionally, the amount thereof to be blended is 0.1 % by weight or more of the total of cosmetics. When the amount of the antibacterial finely divided polymer particle to be blended in cosmetics is below 0.1 % by weight, the resulting antibacterial action is poor.

Because the antibacterial finely divided polymer particle of the invention is highly safe, the amount thereof to be blended is not specifically limited. Generally, the upper limit of the amount is about 50 % by weight of the total of cosmetics. In the cosmetics, further, various cosmetic components for general use can be blended appropriately, including for example oils and fats, surfactants, water-soluble polymers, drugs, dyes, organic powders, perfume, moisturizers, ultraviolet absorbents, preservatives, adsorbents, and gelling agents.

The fats and oils include for example polyvalent alcohols such as glycerin, polyethylene glycol, and propylene glycol, waxes such as beeswax, carnauba wax, lanoline, shellac, microcrystalline wax, and candelilla wax, oils and fats such as sesame seed oil, palm oil, palm seed oil, camellia oil, and olive oil, higher alcohols such as stearyl alcohol, cetyl alcohol, lauryl alcohol, monostearyl glycerin ether, isostearyl alcohol, and octyl dodecanol, esters such as isopropyl myristate, cetyl octate, cetyl lactate, lanolin acetate, and ethyl acetate, and silicone oils such as decamethylcyclopentasiloxane, methylpolysiloxane, and dimethylpolysiloxane, and additionally include fluid paraffin, vaseline, paraffin wax and squalene.

The surfactants include for example higher fatty acid salts (more specifically, potassium salts of fatty acids such as potassium laurate, potassiummyristate, and potassium palmitate, triethanol amine salts prepared by neutralizing fatty acids such as lauric acid, myristic acid and palmitic acid with triethanolamine, and the like), anionic surfactants (N-acyl-L-glutamate sodium, N-lauroyl-L-glutamate sodium, lauroyl-L-glutamate triethanolamine, lauroyl sarcosine sodium, laurylsulfoacetate sodium, laurylsulfate sodium, laurylsulfate triethanolamine, alkyl ether sulfate ester salts, N-acyl sarcosinate salts, N-myristoyl-N-methyltaurine sodium, POE stearyl ether phosphate, POE alkyl ether carboxylic acid, etc.), nonionic surfactants (monolaurate sorbitan, monostearate sorbitan, sucrose fatty acid ester, glycerin fatty acid ester, polyoxyethylene cetyl ether, polyoxyethylene stearyl ether, polyoxyethylene oleyl ether, monostearate polyethylene glycol, monooleate polyoxyethylene sorbitan, monolaurate polyethylene sorbit, POE fatty acid esters, POE lauryl ether, polyoxyethylene-hardened castor oil, etc.), cationic surfactants (cetyl chloride trimethylammonium, stearyl chloride trimethylammonium, stearyl chloride dimethylbenzylammonium, POE alkyl amine, polyamine fatty acid derivatives, amyl alcohol fatty acid derivatives, etc.), and amphoteric surfactants (lauryl dimethylaminoacetate betaine, 2-alkyl-N-carboxymethyl-N-hydroxyethylimidazolium betaine, palm oil fatty acid amide propylbetaine, etc.).

The water-soluble polymers include for example carboxymethyl cellulose, methyl cellulose, hydroxy methyl cellulose, polyvinyl alcohol, polyvinyl pyrrolidone, tragacanth, carrageenan, dextrin, gelatin and gum arabic.

The drugs include for example naturally occurring antibacterial agents and crude drugs such as vitamins, hinokitiol, and chitosan.

The dyes include for example talc, kaolin, silica, mica, sericite, muscovite, phlogopite, lepidolite, biotite and silica.

The organic powders include for example nylon powder, polyethylene powder, polymethacrylate methyl powder and cellulose powder.

The perfume includes for example essential oils of lavender, lemon, lime, jasmine, and rose and animal perfume such as musk.

The moisturizers include sorbitol, xylitol, lactic acid, sodium lactate, glycerin, maltitol, propylene glycol, 1,3-butylene glycol, polyethylene glycol, collagens and hyaluronic acid.

The ultraviolet absorbents include for example p-methoxycinnamate octyl, oxybenzene, urocanic acid, urocanate ethyl, urocanate ethyl ester, oxybenzene sulfonate, tetrahydroxybenzophenone, dihydroxydimethoxybenzophenone, dihydroxybenzophenone, cinoxate, diisopropylcinnamate methyl, methoxycinnamate octyl, p-aminobenzoic acid, p-aminobenzoate ethyl, salicylic acid, salicylate phenyl and salicylate ester.

The preservatives include for example benzoic acid, benzoate salts, p-oxybenzoate ester (paraben), hinokitiol, chitosan, chlorhexidine hydrochloride, benzalkonium chloride, salicylic acid and alcohol.

By blending the antibacterial finely divided polymer particle of the invention in cosmetics, the amount of an organic antibacterial agent to be used can be reduced, so that antibacterial cosmetics with no content of any organic antibacterial agent such as paraben may possibly be provided. Herein, some types of organic antibacterial agents exert effects such as pharmaceutical efficacy other than the antibacterial effect, while simultaneously exerting the antibacterial action on contaminated germs. In such case, the blend and composition should be determined appropriately, taking account of the exertion of such effect.

When p-oxybenzoate ester is blended as an organic antibacterial agent in the cosmetics of the invention, however, p-oxybenzoate ester may potentially modify polymers as described above. In that case, thus, the antibacterial agent is used at less than 1 % by weight, preferably within a range of 0.5 % by weight or less of the total cosmetics.

The adsorbents include for example active charcoal, silica gel, diatomaceous earth, China clay, active alumina, and zeolite.

The gelling agents include for example carboxyvinyl polymer, carboxy polymer salts, alginic acid, alginate salts, acryl · methacrylate alkyl copolymers, acryl methacrylate alkyl copolymer salts, agar, carrageenan, guar gum, gum arabic, tragacanth gum, cellulose, pectin, gelatin, casein, aluminium stearate, and magnesium stearate.

Additionally when the polymer composing the antibacterial finely divided polymer particle of the invention is an acrylic resin such as (meth)acrylic acid-(meth)acrylate ester copolymer and (meth)acrylate ester, the resulting antibacterial finely divided polymer particle is very useful for filling and repairing decayed parts on dental surface and as implanting agents of defected parts.

Acrylic resins such as (meth)acrylic acid-(meth) acrylate ester copolymer and (meth)acrylate ester are frequently used for filling and repairing decayed parts on dental surface and as implanting agents of defected parts. However, oral bacteria are easily deposited and proliferate on the surface of these materials.

Because the antibacterial finely divided polymer particle of the-invention has the antibacterial profile, in contrast, the antibacterial finely divided polymer particle of the invention is used as cosmetics of dental surface, namely for filling and repairing decayed parts or as implanting agents of defected parts, to thereby suppress bacterial deposition and proliferation, so that secondary dental decay and periodontitis can be prevented.

### BEST MODE FOR CARRYING OUT THE INVENTION

The invention is now described in more detail in the following Examples and Comparative Examples. However, the invention is not limited to these Examples. Example

### [Synthesis of inorganic antibacterial agent]

### (1) Hydroxyapatite - silver-based antibacterial agent

100 g of hydroxyapatite and 3.3 g of silver nitrate were added to 1,000 ml of distilled water, for sufficient agitation and mixing at ambient temperature. The resulting product was thoroughly washed in distilled water and was then dried at 120°C wet process: the following preparative methods are the same).

Then, the dried product was calcined at 1,000°C. The resulting calcined product was pulverized, to recover a hydroxyapatite powder with silver immobilized thereon. The mean particle size of the powder was 1.2 µm, while the silver ratio (immobilized amount) was 2.0 % by weight.

Using a sand mill ("Dinomill" under trade name) manufactured by SHINMARU ENTERPRISES CORPORATION, the calcined hydroxyapatite powder was treated, to prepare a finely divided powder to a mean particle size of 0.1 µm.

### (2) Tricalcium phosphate - silver-based antibacterial agent

100 g of tricalcium phosphate and 3.3 g of silver nitrate were added to 1,000 ml of distilled water, for sufficient agitation and mixing at ambient temperature. The resulting product was thoroughly washed in distilled water and was then dried at 120°C.

Then, the dried product was calcined at 700°C. The resulting product was pulverized, to recover a tricalcium phosphate powder with silver immobilized thereon (tricalcium phosphate - silver-based antibacterial agent). The silver ratio (immobilized amount) in the powder was 2.0 % by weight.

Using Dinomill, the calcined tricalcium phosphate was treated for preparing a finely divided powder to a mean particle size of 0.2 µm.

### (3) Zirconium phosphate - silver-based antibacterial agent

100 g of dried zirconium phosphate and 3.3 g of silver nitrate were added to 1,000 ml of distilled water, for sufficient agitation and mixing at ambient temperature. The resulting product was thoroughly washed in distilled water and was then dried at 120°C.

Then, the dried product was pulverized to recover a zirconium phosphate powder with silver immobilized thereon (zirconium phosphate - silver-based antibacterial agent). The silver ratio (immobilized amount) in the powder was 2.0 % by weight.

Using Dinomill, the zirconium phosphate powder was treated for preparing a finely divided powder to a mean particle size of 0.1 µm.

### (4) Titanium oxide - silver-based antibacterial agent

100 g of dried titanium oxide and 3.3 g of silver nitrate were added to 1,000 ml of distilled water, for sufficient agitation and mixing at ambient temperature. The resulting product was thoroughly washed in distilled water and was then dried at 120°C.

Then, the dried product was pulverized, to recover a titanium oxide powder with silver immobilized thereon (titanium oxide - silver-based antibacterial agent). The silver ratio (immobilized amount) in the powder was 2.0 % by weight.

Using Dinomill, the titanium oxide powder was treated for preparing a finely divided powder to a mean particle size of 0.1 µm.

### [Production of antibacterial finely divided nylon particle]

(1) 50 g of ε-caprolactam, 7 g of the antibacterial hydroxyapatite after the treatment for finely divided particle preparation, 200 ml of fluid paraffin and 1 g of soda stearate were mixed together.
(2) Then, the mixture was heated in nitrogen atmosphere at 140°C to dissolve ε-caprolactam, under simultaneous addition of phosphorus trichloride of 0.2 ml as a polymerization accelerator. Then, the resulting mixture was mixed together for about one hour for progressing polymerization, to recover a 6-nylon particle with the antibacterial hydroxyapatite dispersed therein.
(3) After the particle was sieved and washed with added benzene, further, the resulting particle was dried under reduced pressure at 80°C, to recover a 6-nylon particle of a mean particle size of about 8 µm and with the antibacterial hydroxyapatite dispersed therein.

### [Production of antibacterial finely divided acrylic polymer particle]

(1) 1.2 liters of water were placed in a 3-liter four-necked separable flask equipped with an agitator, a reflux cooler and a thermometer, followed by addition of a dispersant polyvinyl alcohol of 16.0 g for dissolution, to prepare a liquid phase. To the liquid phase was then added 15 g of the antibacterial hydroxyapatite after the finely divided particle treatment.
(2) 140 g of ethyl acrylate, 10 g of ethylene glycol dimethacrylate, and 1 g of azobisisobutyronitrile were mixed together, to prepare a uniform oil phase.
(3) Then, the oil phase was added to the aqueous phase. Using a dispersing machine at an agitation velocity of 5,000 rpm, the contents in the flask were dispersed for 3 minutes, to prepare a uniform suspension solution.
(4) Thereafter, the inside-of the reaction system was elevated to 65°C in nitrogen purge under agitation, for 7-hour suspension polymerization. Then, the reaction system was cooled to ambient temperature.
(5) The suspension was filtered to recover the residue, which was then dried to recover a resin particle of a mean particle of 6.7 µm.

### [Production of antibacterial finely divided polymer particle]

### (1) Antibacterial agent-ultraviolet absorbent-ultraviolet reflector-containing nylon particle

100 g of a nylon-12 powder of a mean particle size of 8.5 µm, 15 g of the tricalcium phosphate-silver-based antibacterial agent after the treatment for finely divided particle preparation, 5 g of zinc oxide (mean particle size of 0.1 µm), and 5 g of aluminium oxide (mean particle size of 0.1 µm) were agitated at a high speed with Henschel mixer, to recover a nylon powder containing tricalcium phosphate, zinc oxide, and aluminium oxide (antibacterial agent-ultraviolet absorbent-ultraviolet reflector- containing nylon particle).

### (2) Antibacterial agent-ultraviolet absorbent-pigment- containing acryl particle

100 g of an acryl bead of a mean particle size of 10.3 µm, 20 g of the zirconium phosphate - silver-based antibacterial agent after the treatment for finely divided particle preparation, 10 g of titanium oxide (mean particle size of 0.1 µm), and 5 g of lepidolite (mean particle size of 0.3 µm) were agitated at a high speed with Henschel mixer, to recover an acryl particle containing zirconium phosphate, titanium oxide, and lepidolite (antibacterial agent-ultraviolet absorbent-pigment-containing acryl particle).

### (3) Antibacterial agent-pigment- containing acryl particle

100 g of an acryl bead of a mean particle size of 10.3 µm, 20 g of the titanium oxide after the treatment for finely divided particle preparation, and 10 g of lepidolite (mean particle size of 0.3 µm) were agitated with a hybridizer at a high speed, to recover an acryl particle containing titanium oxide and lepidolite (antibacterial agent-pigment-containing acryl particle).

### [Cosmetic composition]

| (Cake-type eyeliner) | |
|---|---|
| 1. The antibacterial finely divided nylon particle of the invention | 37.0 % by weight |
| 2. White Vaseline | 4.0 % by weight |
| 3. Beeswax | 5.0 % by weight |
| 4. Carnauba wax | 1.0 % by weight |
| 5. Microcrystalline wax | 6.0 % by weight |
| 6. Fluid paraffin | 28.8 % by weight |
| 7. Stearate monoglycerin ester | 1.0 % by weight |
| 8. Squalene | 12.0 % by weight |
| 9. Ultramarine blue | 5.0 % by weight |
| 10. Perfume | 0.2 % by weight |

Process: Among the components, white vaseline, beeswax, carnauba wax, microcrystalline wax, fluid paraffin, stearate monoglycerin ester and squalene were mixed together and dissolved together under heating, followed by addition and dispersion of the antibacterial finely divided nylon particle of the invention, ultramarine blue and perfume to the resulting mixture. Then, the resulting mixture was filled in a mold at 80°C and was cooled therein, to recover a cake-type eyeliner.

| (Power foundation) | |
|---|---|
| 1. The antibacterial finely divided acrylic polymer particle of the invention | 35.0 % by weight |
| 2. Mica | 20.0 % by weight |
| 3. Talc | 10.0 % by weight |
| 4. Titanium oxide | 10.0 % by weight |
| 5. Zinc oxide | 5.0 % by weight |
| 6. Red oxide | 3.0 % by weight |
| 7. Squalene | 5.0 % by weight |
| 8. Myristate isotridodecyl | 2.5 % by weight |
| 9. Fluid paraffin | 9.3 % by weight |
| 10. Perfume | 0.2 % by weight |

Process: Among the components, the antibacterial finely divided acrylic polymer particle of the invention, mica, talc, titanium oxide, zinc oxide and red oxide are mixed together, and the resulting mixture is then disrupted through a pulverizer. This is transferred to a high-speed blender, followed by addition and mixing of squalene and the following components, to prepare a uniform mixture. This is subsequently treated with a pulverizer and is passed through a sieve to adjust the particle size to a given value, which is then filled in a container, where the particle is compressed and molded.

| (Cream) | |
|---|---|
| 1. The antibacterial finely divided nylon particle of the invention | 12.0 % by weight |
| 2. Solid paraffin | 5.0 % by weight |
| 3. Vaseline | 14.0 % by weight |
| 4. Sorbitan monooleate | 5.0 % by weight |
| 5. Polyoxyethylene sorbitan monooleate | 2.0 % by weight |
| 6. Fluid paraffin | 35.0 % by weight |
| 7. Distilled water | 26.5 % by weight |
| 8. Perfume | 0.5 % by weight |

Process: Distilled water was heated and kept at 70°C, to which were then gradually added the remaining components except for the antibacterial finely divided nylon particle and perfume, for preliminary emulsification. The resulting mixture was uniformly emulsified with a homomixer, followed by mixing of the antibacterial finely divided nylon particle of the invention and perfume under cooling, to prepare cream.

| (Shampoo) | |
|---|---|
| 1. The antibacterial finely divided nylon particle of the invention | 1.0 % by weight |
| 2. Lactic acid | 0.1 % by weight |
| 3. Alkylsulfate triethanolamine | 15.0 % by weight |
| 4. Palm oil fatty acid monoethanolamide | 2.0 % by weight |
| 5. Ethylene glycol monostearate | 5.0 % by weight |
| 6. Perfume | 0.5 % by weight |
| 7. Distilled water | 76.4 % by weight |

Process: The components were uniformly mixed together and agitated, while the components were heated to 86°C, to prepare the shampoo of the invention.

| (Rinse) | |
|---|---|
| 1. The antibacterial finely divided nylon particle of the invention | 0.5 % by weight |
| 2. Stearyl chloride dimethylbenzylammonium | 1.4 % by weight |
| 3. Stearyl alcohol | 0.6 % by weight |
| 4. Glycerin monostearate | 1.5 % by weight |
| 5. Propylene glycol | 5.0 % by weigh |
| 6. Glycerin | 3.0 % by weigh |
| 7. Distilled water | 88.0 % by weigh |

Process: The components were uniformly mixed and agitated together, while the components were heated to 86°C, to prepare the rinse of the invention.

| (Cleansing cream) | |
|---|---|
| 1. N-Lauroyl-L-glutamate potassium | 10.0 % by weight |
| 2. Potassium stearate | 5.0 % by weight |
| 3. Potassium myristate | 5.0 % by weight |
| 4. The antibacterial finely divided acrylic polymer particle | 5.0 % by weight |
| 5. 1,3-Butylene glycol | 20.5 % by weight |
| 6. Glycerin | 5.0 % by weight |
| 7. Distilled water | 49.5 % by weight |

Process: The components were uniformly mixed and agitated together, while the components were heated to 80°C, to prepare the body shampoo of the invention.

| (Body shampoo) | |
|---|---|
| 1. M-Acyl-L-glutamate sodium | 10.0 % by weight |
| 2. N-Lauroyl-L-glutamate potassium | 5.0 % by weight |
| 3. Potassium myristate | 10.0 % by weight |
| 4. Potassium Laurate | 5.0 % by weight |
| 5. Propylene glycol | 3.0 % by weight |
| 6. The antibacterial finely divided nylon | |
| particle of the invention | 5.0 % by weight |
| 7. Distilled water | qs. |

Process : Among the components, distilled water was heated to 70°C, followed by sequential addition of the remaining components, for sufficient agitation and mixing. The resulting mixture was cooled to ambient temperature, to prepare the body shampoo of the invention.

| (Hair shampoo) | |
|---|---|
| 1. The antibacterial finely divided nylon particle of the invention | 2.0 % by weight |
| 2. Alkyl ether sulfate sodium | 18.0 % by weight |
| 3. Palm oil fatty acid diethanolamide | 2.0 % by weight |
| 4. Cation-modified cellulose ether | 1.5 % by weight |
| 5. Distilled water | qs. |

Process: The components were uniformly mixed and agitated together, while the components were heated to 70°C, to prepare the shampoo of the invention.

| (Hair rinse) | |
|---|---|
| 1. Squalene | 2.0 % by weight |
| 2. Monooleate sorbitan | 2.0 % by weight |
| 3. Cetyl chloride trimethylammonium | 1.5 % by weight |
| 4. Cetanol | 1.0 % by weight |
| 5. Shellac | 0.5 % by weight |
| 6. Monooleate polyoxyethylene | |
| sorbitan | 0.2 % by weight |
| 7. Propylene glycol | 3.0 % by weight |
| 8. Polyvinyl pyrrolidone | 1.0 % by weight |
| 9. The antibacterial finely divided nylon particle of the invention | 1.0 % by weight |
| 10. Distilled water | qs. |
| 11. Perfume | 0.1 % by weight |

Process: The components 1 through 6 were uniformly dissolved and mixed together at about 70°C. Under agitation, the resulting mixture was mixed with the components 7 through 10 uniformly dissolved and mixed together at about 70°C. The resulting mixture was continuously mixed together while the mixture was spontaneously cooled, followed by addition of perfume at about 50°C. Under further mixing, the mixture was cooled spontaneously to ambient temperature, to prepare the hair rinse of the invention.

| (Powder foundation) | |
|---|---|
| 1. Sericite | 10.0 % by weight |
| 2. Mica | 6.5 % by weight |
| 3. The antibacterial finely divided nylon particle of the invention | 50.0 % by weight |
| 4. Titanium oxide | 5.0 % by weight |
| 5. Yellow iron oxide | 1.5 % by weight |
| 6. Red oxide | 0.4 % by weight |
| 7. Black iron oxide | 0.2 % by weight |
| 8. Spherical silica (mean particle size of 0.5 µm) | 16.4 % by weight |
| 9. Methyl polysiloxane | 10.0 % by weight |

Process: The powder components were pulverized and mixed together with a pulverizer, followed by addition of methyl polysiloxane. The resulting mixture was additionally mixed together. This mixture was filled in a medium-size dish, to prepare a solid foundation.

| (Shaving foam) | |
|---|---|
| 1. The antibacterial finely divided nylon particle of the invention | 10.0 % by weight |
| 2. Triethanolamine | 5.0 % by weight |
| 3. Sodium laurate | 8.0 % by weight |
| 4. Sodium myristate | 8.0 % by weight |
| 5. Polyoxyethylene-hardened castor oil | 1.0 % by weight |
| 6. Propellant (isobutane) | 20.0 % by weight |
| 7. Distilled water | qs. |

Process: Among the various components, the remaining components except for the propellant were mixed together. The mixture was filled along with the propellant in a pressure-resistant container, to prepare the shaving foam of the invention as an aerosol product.

| (Face powder) | |
|---|---|
| 1. Talc | 20.0 % by weight |
| 2. Titanium oxide | 0.5 % by weight |
| 3. Red oxide | 0.1 % by weight |
| 4. The antibacterial finely divided nylon particle of the invention | 5.0 % by weight |
| 5. Spherical nylon (mean particle size of 5.0 µm) | 40.0 % by weight |
| 6. Spherical silica (mean particle size of 0.5 µm) | 17.0 % by weight |
| 7. Barium sulfate | 16.0 % by weight |
| 8. Methylpolysiloxane | 1.4 % by weight |

Process: The powder components were pulverized and mixed together with a pulverizer, followed by addition of methylpolysiloxane. The resulting mixture was additionally mixed together. This mixture was passed through a sieve, to prepare a face powder.

| (Lipstick) | |
|---|---|
| 1. Microcrystalline wax | 5.0 % by weight |
| 2. Ceresin | 7.0 % by weight |
| 3. Candelilla wax | 3.0 % by weight |
| 4. Carnauba wax | 5.0 % by weight |
| 5. Myristate octyl decyl | 10.0 % by weight |
| 6. Dimethylpolysiloxane | 12.0 % by weight |
| 7. Castor oil | 30.5 % by weight |
| 8. Fluid paraffin | 15.0 % by weight |
| 9. Titanium mica | 5.0 % by weight |
| 10. The antibacterial finely divided | |
| nylon particle of the invention | 6.0 % by weight |
| 11. Red No. 204 (mean particle size of 0.2 µm) | 1.0 % by weight |
| 12. Yellow No.4 aluminium lake | 0.5 % by weight |

Process: Among the components, the oil components microcrystalline wax through fluid paraffin were mixed together and dissolved under heating to 80 to 90°C. To the resulting mixture was added a uniform mixture of the powder components titanium mica through Yellow No.4 aluminium lake. The mixture was then passed through a roll mill, for dispersion. Then, the dispersion was again dissolved and defoamed, and was then injected and filled in a resin pipe, followed by cooling. Subsequently, the core was pushed out and filled in a container, to prepare the lipstick of the invention.

| [Cosmetic composition] | |
|---|---|
| (Emulsion) | |
| 1. Oxybenzene sulfonate | 1.0 % by weight |
| 2. Cetanol | 1.5 % by weight |
| 3. Vaseline | 1.5 % by weight |
| 4. Hardened palm oil | 1.5 % by weight |
| 5. Beeswax | 1.5 % by weight |
| 6. Squalene | 4.5 % by weight |
| 7. Sesquioleate sorbitan | 2.0 % by weight |
| 8. Antibacterial agent-ultraviolet absorbent-ultraviolet reflector-containing | |
| nylon particle | 2.0 % by weight |
| 9. Polyoxyethylene oleyl ether | 2.5 % by weight |
| 10. Glycerin | 3.0 % by weight |
| 11. Carboxyvinyl polymer | 0.2 % by weight |
| 12. Potassium hydroxide | 0.3 % by weight |
| 13. Perfume | 0.1 % by weight |
| 14. Distilled water | qs. |

Process: Under agitation, the components 1 through 7 dissolved under heating at 80°C were added to 9 through 12 and 14 dissolved under heating at 80°C, and the resulting mixture was sufficiently mixed together. Then, the mixture was cooled under agitation, followed by addition of a nylon particle (component 8) containing the antibacterial agent of the invention, the ultraviolet absorbent and the ultraviolet reflector, for sufficient additional agitation. After further cooling, perfume was added to prepare the emulsion of the invention.

| (Sun screening cream) | |
|---|---|
| 1. p-Methoxycinnamate octyl | 2.0 % by weight |
| 2. Ceresin | 7.0 % by weight |
| 3. Fluid paraffin | 33.0 % by weight |
| 4. Myristate octyl dodecyl | 20.0 % by weight |
| 5. Oleyl alcohol | 12.0 % by weight |
| 6. Sesame seed oil | 10.0 % by weight |
| 7. Antibacterial agent-ultraviolet absorbent-ultraviolet reflector-containing | |
| nylon particle | 15.0 % by weight |
| 8. Perfume | 1.0 % by weight |

Process: The components 1 through 6 were dissolved under heating at 80°C, and were then uniformly mixed together. After cooling, the components 7 and 8 were added to the resulting mixture, which was then uniformly mixed together with a roll mill, and was then filled in a container, to prepare a sun screening cream.

| (Sun screening cream) | |
|---|---|
| 1. Fluid paraffin | 34.0 % by weight |
| 2. Cetanol | 1.5 % by weight |
| 3. Shellac | 5.2 % by weight |
| 4. Stearate polyoxyethylene | |
| stearyl ether | 1.2 % by weight |
| 5. Monostearate sorbitan | 2.0 % by weight |
| 6. Sodium hydroxide | 0.1 % by weight |
| 7. Distilled water | 30.8 % by weight |
| 8. Glycerin | 7.0 % by weight |
| 9. Antibacterial agent-ultraviolet absorbent-ultraviolet reflector-containing nylon particle | 18.0 % by weight |
| 10. Perfume | 0.2 % by weight |

Process: The components 1 through 5 were dissolved under heating at 80°C, while the components 6 and 7 were dissolved under heating at 75°C. These components were mixed together and emulsified, followed by addition of the components 8 to 10 at 60°C. The resulting mixture was sufficiently mixed together and was then mixed together under spontaneous cooling to 30°C or less.

| (Emulsion type sun screening cream) | |
|---|---|
| 1. Octamethylcyclotetrasiloxane | 20.0 % by weight |
| 2. Dimethylpolysiloxane | 10.0 % by weight |
| 3. Dimethylsiloxane methylcetyloxysiloxane copolymer | 1.0 % by weight |
| 4. p-Methoxycinnamate octyl | 5.0 % by weight |
| 5. Antibacterial agent-ultraviolet absorbent-pigment-containing acryl particle | 20.0 % by weight |
| 6. Ethanol | 10.0 % by weight |
| 7. Glycerin | 2.0 % by weight |
| 8. Distilled water | 31.9 % by weight |
| 9. Perfume | 0.1 % by weight |

Process: The components 1 through 4 were dissolved at ambient temperature, followed by addition of the component 5, for dispersion with a disper. To the resulting dispersion were added the following components 6 through 8 under agitation for emulsification. Then, perfume as the component 9 was added to the resulting emulsion, to prepare an emulsion type sun screening cream.

| (Amphibious powder foundation) | |
|---|---|
| 1. Urocanic acid | 3.0 % by weight |
| 2. Phenyl salicylate | 0.2 % by weight |
| 3. Dimethylpolysiloxane | 5.0 % by weight |
| 4. Squalene | 5.0 % by weight |
| 5. Lanoline | 1.0 % by weight |
| 6. Vaseline | 1.0 % by weight |
| 7. Fluid paraffin | 1.0 % by weight |
| 8. Glycerin | 3.8 % by weight |
| 9. Perfume | 0.1 % by weight |
| 10. The antibacterial agent-ultraviolet absorbent-pigment-containing acryl particle | 79.9 % by weight |

Process: The components 1 through 9 were mixed together under heating to preliminarily prepare a uniform mixture. Then, the component 10 was placed in a high-speed blender, to which the resulting mixture was added for uniform mixing. The mixture was taken out and treated with a pulverizer, and was then passed through a sieve to adjust the particle size to a given value. Then, the particle was press molded in a mold, to prepare the amphibious powder foundation of the invention.

| (Sunscreen oil) | |
|---|---|
| 1. Dimethylpolysiloxane | 30.0 % by weight |
| 2. Fluid paraffin | 30.0 % by weight |
| 3. Decamethylcyclopentasiloxane | 19.0 % by weight |
| 4. Microcrystalline wax | 1.0 % by weight |
| 5. p-Aminobenzoic acid | 5.0 % by weight |
| 6. Amylsalicylate | 5.0 % by weight |
| 7. Octyl methoxycinnamate | 5.0 % by weight |
| 8. Antibacterial agent-pigment-containing acryl particle | 5.0 % by weight |

Process: The components 1 through 7 were dissolved and mixed together uniformly at 70°C, and then, the component 8 was blended and dispersed therein and cooled at ambient temperature, to prepare the sunscreen oil of the invention.

| (Two-layer separation type sunscreen emulsion) | |
|---|---|
| 1. Decamethylcyclopentasiloxane | 20.0 % by weight |
| 2. Dimethylpolysiloxane | 20.0 % by weight |
| 3. Dimethylsiloxane methylcetyloxysiloxane copolymer | 1.0 % by weight |
| 4. Dihydroxydimethoxybenzophenone | 2.0 % by weight |
| 5. Antibacterial agent-pigment-containing acryl particle | 18.0 % by weight |
| 6. Distilled water | qs. |
| 7. 1,3-Butylene glycol | 5.0 % by weight |
| 8. Ethanol | 5.0 % by weight |
| 9. Perfume | 0.1 % by weight |

Process: The components 1 through 4 were dissolved at ambient temperature, followed by addition of the component 5 for dispersion treatment. To the resulting dispersion were added the components 6 through 8 under agitation, for emulsification. Then, the perfume 9 was added to the resulting emulsion, to prepare the two-layer separation type sunscreen emulsion of the invention.

| (Emulsion type sun screening cream) | |
|---|---|
| 1. Stearic acid | 2.5 % by weight |
| 2. White beeswax | 1.5 % by weight |
| 3. Microcrystalline wax | 2.0 % by weight |
| 4. Squalene | 10.0 % by weight |
| 5. Fluid paraffin | 7.0 % by weight |
| 6. Glycerin monostearate | 3.0 % by weight |
| 7. Antibacterial agent-pigment-containing acryl particle | 6.0 % by weight |
| 8. Glycerin | 12.0 % by weight |
| 9. Triethanolamine | 1.0 % by weight |
| 10. Distilled water | qs. |
| 11. Perfume | 0.1 % by weight |

Process: The components 1 through 6 as mixed together under heating at 70°C were added to the components 7 through 10 as mixed together under heating at 70°C. The resulting mixture was sufficiently mixed together with a homomixer. The mixture was continuously mixed together under spontaneous cooling, followed by addition of perfume around 50°C. Then, agitation continued until the temperature reached ambient temperature, to prepare the emulsion type sun screening cream of the invention.

### [Antibacterial test 1]

The antibacterial effects of the Examples, Comparative Examples and Control Example of the compositions in Table 1 were evaluated.

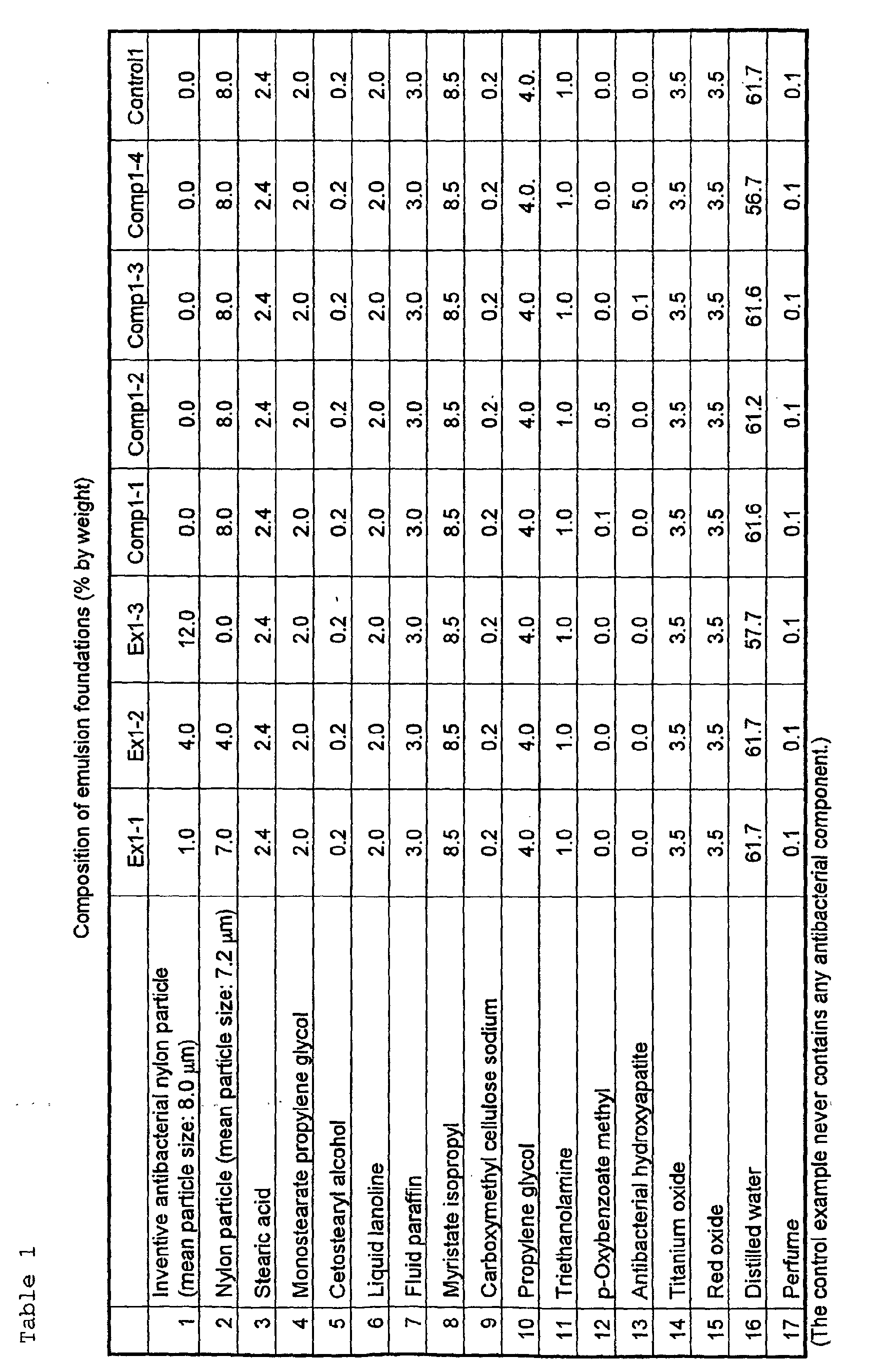

### (1) Production process of cosmetic composition (emulsion foundation)

The oil phase components (3) through (8) in Table 1 were mixed together and heated to 75°C, for uniform preparation. Meanwhile, the components (1) and (2) and the components (9) through (16) were uniformly dispersed with a homomixer, and were then kept at 75°C, which were then prepared into the form of an aqueous phase. To the oil phase was added the aqueous phase, for emulsification dispersion. The resulting dispersion was cooled to 30°C, followed by addition of the component (17) for mixing.

Additionally, the antibacterial hydroxyapatite (the mean particle size of 1.2 µm; the amount thereof to be immobilized was 2.0 % by weight) used for the production of the antibacterial finely divided nylon particle of the invention was used.

### (2) Procedure of antibacterial test

As bacteria, Escherichia coli and Staphylococcus aureus were used. As fungus, Aspergillus niger was used. 10⁶ bacterial cells or 10⁵ fungal cells were inoculated per one gram sample, for culturing at 37°C, to count viable microbial cells two weeks later.

### (3) Evaluation of antibacterial effect

The results of the antibacterial test are shown in Table 2.

**Table 2**

| Viable microbial cells two weeks later (cells/g) | | | |
|---|---|---|---|
| | Escherichia coli | Staphylococcus aureus | Aspergillus niger |
| Example 1-1 | 1.2 × 10 | 3.2 × 10 | 8.5 × 10 |
| Example 1-2 | 0 | 2.1 × 10 | 3.8 × 10 |
| Example 1-3 | 0 | 0 | 0 |
| Comparative Example 1-1 | 8.6 × 10² | 7.2 × 10² | 6.5 × 10³ |
| Comparative Example 1-2 | 2.3 × 10² | 1.7 × 10² | 1.3 × 10³ |
| Comparative Example 1-3 | 4.8 × 10³ | 3.1 × 10³ | 2.5 × 10⁴ |
| Comparative Example 1-4 | 4.1 × 10² | 3.3 × 10² | 3.1 × 10³ |
| Control Example 1 | 1.2 × 10⁶ | 1.1 × 10⁶ | 1.2 × 10⁵ |

As apparently shown in the table, the Examples of the invention, where the antibacterial nylon beads were used, exerted satisfactory effects on both the bacteria and the fungus. In contrast, Comparative Example 1-1 and Comparative Example 1-2 using p-oxybenzoate methyl as the antibacterial agent could not exert satisfactory antibacterial effect.

Alternatively, satisfactory antibacterial effect could not be observed in Comparative Example 1-3 and Comparative Example 1-4, where the antibacterial hydroxyapatite was not contained in the nylon beads but the antibacterial hydroxyapatite and the nylon beads were separately contained.

### (Evaluation of applicability)

### (1) The emulsion foundations of Example 1-1 through 1-3, Comparative Examples 1-1 through 1-4, and Control Example were evaluated of the applicability during use in terms of three items, namely adhesiveness to skin, spreadability on skin and smoothness during use.

The evaluation of the applicability during use was made by allowing female 20 panelists from age 20 to age fifties to use the Examples, Comparative Examples and Control Example and rank them according to the evaluation standards shown in Table 3.

**Table 3**

| Evaluation standards for applicability | | |
|---|---|---|
| Items evaluated for applicability | Evaluation | Evaluated score |
| Adhesiveness to skin | Good | 5 |
| | More or less good | 4 |
| | Normal | 3 |
| | More or less bad | 2 |
| | Bad | 1 |
| Spreadability on skin | Good | 5 |
| | More or less good | 4 |
| | Normal | 3 |
| | More or less bad | 2 |
| | Bad | 1 |
| Smoothness during use | Good | 5 |
| | More or less good | 4 |
| | Normal | 3 |
| | More or less bad | 2 |
| | Bad | 1 |

The evaluation results are expressed as follows according to the 4-grade evaluation by 20 females.
Double circle: 4.5 to 5.0
Circle: 3.5 to less than 4.5
Triangle: 2.5 to less than 3.5
x: less than 2.5

### (2) The results of the evaluation of the applicability during use are shown in Table 4.

**Table 4**

| Results of evaluation of the applicability during use | | | |
|---|---|---|---|
| | Adhesiveness to skin | Spreadability on skin | Smoothness during use |
| Example 1-1 | Double circle | Double circle | Double circle |
| Example 1-2 | Double circle | Double circle | Double circle |
| Example 1-3 | Double circle | Double circle | Double circle |
| Comparative Example 1-1 | Circle | Circle | Circle |
| Comparative Example 1-2 | × | Triangle | × |
| Comparative Example 1-3 | Circle | Circle | Triangle |
| Comparative Example 1-4 | Triangle | Triangle | × |
| Control Example 1 | Circle | Circle | Circle |

As apparently shown in the table, all of the inventive emulsion foundations had good applicability. In contract, the evaluated applicability concerning Comparative Examples 1-1 through 1-4 are poor, compared with those of the Examples.

Comparative Example 1-1 and Comparative Example 1-2 contain p-oxybenzoate methyl as the component, so the component may deteriorate the nylon beads.

Further, the antibacterial hydroxyapatite is blended as the component in Comparative Example 1-3 and Comparative Example 1-4, and sometimes causes aggregation, so that the applicability during use may be deteriorated.

### [Antibacterial test 2]

The antibacterial effects of the Examples, Comparative Examples and Control Example of the compositions in Table 5 were evaluated.

**Table 5**

| Composition of lipstick components (% by weight) | | | | | | | | |
|---|---|---|---|---|---|---|---|---|
| | | Example | | | Comparative Example | | | Control Example |
| | | 2-1 | 2-2 | 2-3 | 2-1 | 2-2 | 2-3 | 2 |
| 1 | Inventive antibacterial nylon particle (mean particle size: 8.0 µm) | 1.0 | 5.0 | 10.0 | 0.0 | 0.0 | 0.0 | 0.0 |
| 2 | Nylon particle (mean particle size: 7.2 µm) | 5.0 | 1.0 | 0.0 | 5.5 | 5.4 | 5.0 | 6.0 |
| 3 | Microcrystalline wax | 5.0 | 5.0 | 5.0 | 5.0 | 5.0 | 5.0 | 5.0 |
| 4 | Ceresin | 5.0 | 5.0 | 5.0 | 5.0 | 5.0 | 5.0 | 5.0 |
| 5 | Candelilla wax | 15.0 | 15.0 | 15.0 | 15.0 | 15.0 | 15.0 | 15.0 |
| 6 | Carnauba wax | 5.0 | 5.0 | 5.0 | 5.0 | 5.0 | 5.0 | 5.0 |
| 7 | Myristate octyl decyl | 10.0 | 10.0 | 8.0 | 10.0 | 10.0 | 10.0 | 10.0 |
| 8 | Dimethylpolysiloxane | 12.0 | 12.0 | 10.0 | 12.0 | 12.0 | 12.0 | 12.0 |
| 9 | Castor oil | 15.5 | 15.5 | 15.5 | 15.5 | 15.5 | 15.5 | 15.5 |
| 10 | Fluid paraffin | 20.0 | 20.0 | 20.0 | 20.0 | 20.0 | 20.0 | 20.0 |
| 11 | Titanium mica | 5.0 | 5.0 | 5.0 | 5.0 | 5.0 | 5.0 | 5.0 |
| 12 | p-Oxybenzoate methyl | 0.0 | 0.0 | 0.0 | 0.0 | 0.1 | 0.5 | 0.0 |
| 13 | Antibacterial tricalcium phosphate | 0.0 | 0.0 | 0.0 | 0.5 | 0.5 | 0.5 | 0.0 |
| 14 | Red No. 204 (mean particle size: 0.2 µm) | 1.0 | 1.0 | 1.0 | 1.0 | 1.0 | 1.0 | 1.0 |
| 15 | Yellow No.4 aluminium lake | 0.5 | 0.5 | 0.5 | 0.5 | 0.5 | 0.5 | 0.5 |
| (The control example never contains any antibacterial component.) | | | | | | | | |

### (1) Production process of cosmetic composition (lipstick)

Among the components shown in Table 5, the oil phase components microcrystalline wax through fluid paraffin (the components (3) through (10) in Table 5) were mixed together and solubilized under heating to 80°C, to which was added a uniform mixture of the components titanium mica through Yellow No.4 aluminium lake (the components (11) through (15) in Table 5) and was also added if necessary a uniform mixture of the components (1) and (2). The resulting mixture passed through a roll mill, for dispersion. After the resulting dispersion was again solubilized and defoamed, the dispersion was injected and filled in a resin pipe, and cooled. Subsequently, the core was extruded and filled in a container, to prepare the object cosmetic (lip stick).

Herein, the "tricalcium phosphate-silver-based antibacterial agent" produced according to the formulation (2) in the [Synthesis of inorganic antibacterial agent] was used as the inorganic antibacterial agent contained in the "antibacterial finely divided nylon particle of the invention" as the component (1) in Table 5.

### (2) Procedure of antibacterial test

As bacteria, Escherichia coli and Staphylococcus aureus were used. As fungus, Aspergillus niger was used. 10⁶ bacterial cells or 10⁵ fungal cells were inoculated per one gram sample, for culturing at 37°C, to count viable microbial cells two weeks later.

**Table 6**

| Viable microbial cells two weeks later (cells/g) | | | |
|---|---|---|---|
| | Escherichia coli | Staphylococcus aureus | Aspergillus niger |
| Example 2-1 | 1.1 × 10 | 3.3 × 10 | 8.4 × 10 |
| Example 2-2 | 0 | 2.3 × 10 | 3.9 × 10 |
| Example 2-3 | 0 | 0 | 0 |
| Comparative Example 2-1 | 7.9 × 10² | 7.4 × 10² | 6.4 × 10³ |
| Comparative Example 2-2 | 3.9 × 10² | 3.1 × 10² | 3.0 × 10³ |
| Comparative Example 2-3 | 2.3 × 10² | 1.8 × 10² | 1.2 × 10³ |
| Control Example 1 | 1.2 × 10⁶ | 1.1 × 10⁶ | 1.2 × 10⁵ |

As apparently shown in the table, the Examples of the invention, where the antibacterial nylon beads were used, exerted satisfactory effects on both the bacteria and the fungus. In contrast, Comparative Example 2-2 and Comparative Example 2-3 using p-oxybenzoate methyl as the antibacterial agent could not exert satisfactory antibacterial effect.

Alternatively, satisfactory antibacterial effect could not be observed in Comparative Examples 2-1 through 2-3, where the antibacterial tricalcium phosphate was contained as the antibacterial component.

### [Antibacterial test 3]

The antibacterial effects of the Examples, Comparative Examples and Control Example of the compositions in Table 7 were evaluated.

**Table 7**

| Composition of sun screening cream (% by weight) | | | | | | | | |
|---|---|---|---|---|---|---|---|---|
| | | Example | | | Comparative Example | | | Control Example |
| | | 3-1 | 3-2 | 3-3 | 3-1 | 3-2 | 3-3 | 3 |
| 1 | antibacterial agent-ultraviolet absorbent-ultraviolet reflector-containing nylon particle (mean particle size: 8.0 µm) | 0.3 | 0.5 | 15.0 | 0.01 | 0.02 | 0.05 | 0.0 |
| 2 | Nylon particle (mean particle size: 7.2 µm) | 8.0 | 8.0 | 0.0 | 5.5 | 5.5 | 5.5 | 5.5 |
| 3 | Fluid paraffin | 34.0 | 34.0 | 34.0 | 34.0 | 34.0 | 34.0 | 34.0 |
| 4 | Cetanol | 1.5 | 1.5 | 1.5 | 1.5 | 1.5 | 1.5 | 1.5 |
| 5 | Shellac | 5.2 | 5.2 | 5.2 | 5.2 | 5.2 | 5.2 | 5.2 |
| 6 | Stearate polyoxyethylene stearyl ether | 1.2 | 1.2 | 1.2 | 1.2 | 1.2 | 1.2 | 1.2 |
| 7 | Monostearate sorbitan | 2.0 | 2.0 | 2.0 | 2.0 | 2.0 | 2.0 | 2.0 |
| 8 | Sodium hydroxide | 0.1 | 0.1 | 0.1 | 0.1 | 0.1 | 0.1 | 0.1 |
| 9 | Distilled water | 40.5 | 40.3 | 33.8 | 42.79 | 42.68 | 42.25 | 43.3 |
| 10 | Glycerin | 7.0 | 7.0 | 7.0 | 7.0 | 7.0 | 7.0 | 7.0 |
| 11 | Perfume | 0.2 | 0.2 | 0.2 | 0.2 | 0.2 | 0.2 | 0.2 |
| 12 | p-Oxybenzoate methyl | 0.0 | 0.0 | 0.0 | 0.0 | 0.1 | 0.5 | 0.0 |
| 13 | Antibacterial tricalcium phosphate | 0.0 | 0.0 | 0.0 | 0.5 | 0.5 | 0.5 | 0.0 |
| (The control example never contains any antibacterial component.) | | | | | | | | |

### (1) Production process of cosmetic composition(sun screening cream)

Among the components shown in Table 7, the components fluid paraffin through monostearate sorbitan (the components (3) through (7) in Table 7) were solubilized under heating to 80°C. Alternatively, sodium hydroxide (the component (8) in Table 7) and distilled water (the component (9) in Table 7) were solubilized under heating at 75°C. These components were mixed together and emulsified. While keeping the mixture at 60°C, the components (10) through (13), (1) and (2) in Table 7 were added to the mixture for sufficient mixing, which was then mixed together under spontaneous cooling until 30°C.

Herein, the "antibacterial agent-ultraviolet absorbent-ultraviolet reflector-containing nylon particle" (simply abbreviated as "the antibacterial agent of the invention" hereinafter) produced according to the formulation (1) in the [Synthesis of antibacterial finely divided polymer particle] was used as the "antibacterial agent-ultraviolet absorbent-ultraviolet reflector-containing nylon particle" as the component (1) in Table 7.

### (2) Procedure of antibacterial test

As bacteria, Escherichia coli and Staphylococcus aureus were used. As fungus, Aspergillus niger was used. 10⁶ bacterial cells or 10⁵ fungal cells were inoculated per one gram sample, for culturing at 37°C, to count viable microbial cells two weeks later.

**Table 8**

| Viable microbial cells two weeks later (cells/g) | | | |
|---|---|---|---|
| | Escherichia coli | Staphylococcus aureus | Aspergillus niger |
| Example 3-1 | 5.1 × 10 | 6.5 × 10 | 1.1 × 10² |
| Example 3-2 | 2.5 × 10 | 5.1 × 10 | 6.8 × 10 |
| Example 3-3 | 0 | 0 | 0 |
| Comparative Example 3-1 | 8.1 × 10² | 8.1 × 10² | 7.1 × 10³ |
| Comparative Example 3-2 | 4.2 × 10² | 3.2 × 10² | 4.1 × 10³ |
| Comparative Example 3-3 | 2.9 × 10² | 2.2 × 10² | 1.7 × 10³ |
| Control Example 3 | 1.4 × 10⁶ | 1.4 × 10⁶ | 2.1 × 10⁵ |

As apparently shown in the table, the Examples of the invention, where the antibacterial agent of the invention was used, exerted satisfactory effects on both the bacteria and the fungus. In contrast, Comparative Example 3-2 and Comparative Example 3-3 using p-oxybenzoate methyl as the antibacterial agent could not exert satisfactory antibacterial effect.

Alternatively, satisfactory antibacterial effect could not be observed in Comparative Examples 3-1 through 3-3, where tricalcium phosphate was contained as the antibacterial component.

## Claims

1. An antibacterial resin for cosmetics which contains an inorganic antibacterial agent, **characterized in that** the antibacterial resin comprises spherical particles of a mean particle size of 0.1 µm to 1,000 µm.

2. An antibacterial resin according to claim 1, **characterized in that** the resin comprises a synthetic polymer.

3. An antibacterial resin according to claim 1, wherein the content of the inorganic antibacterial agent is 0.01 to 30 % by weight.

4. An antibacterial resin according to claim 1, **characterized in that** at least one antibacterial metal selected from the group consisting of silver, copper, zinc, gold, platinum and nickel being immobilized on at least one ceramics selected from the group consisting of alumina, silica, zeolite, phosphate salt-series compounds, calcium carbonate, calcium silicate, bentonite, and titanium oxide is used as the inorganic antibacterial agent.

5. An antibacterial resin according to claim 1, wherein the resin is polyamide.

6. An antibacterial resin according to claim 1, **characterized in that** the resin is methacrylate ester polymer.

7. A method for producing an antibacterial resin for cosmetics according to claim 1, **characterized by** adding an inorganic antibacterial agent to a monomer component of a resin and subjecting the resulting mixture to suspension polymerization.

8. A method for producing an antibacterial resin according to claim 1, **characterized by** mixing an inorganic antibacterial agent with a monomer component of a resin during suspension polymerization.

9. An antibacterial resin according to any one of claims 1 through 6, **characterized in that** the mean particle size of the inorganic antibacterial agent is 1/20-fold to 1/2-fold the mean particle size of an antibacterial resin containing the inorganic antibacterial agent.

10. An antibacterial resin **characterized by** containing an inorganic antibacterial agent as the essential component and additionally containing at least one component selected from an ultraviolet reflector, an ultraviolet absorbent, a pigment, a lipid adsorbent, a moisturizer, and a water-repellent oil-repellent substance.

11. A cosmetic **characterized by** blending an antibacterial resin according to any one of claims 1 through 6, claim 9 and claim 10.
